# EUROPÄISCHE PATENTANMELDUNG

(11) **EP 1 205 470 A2**
(43) Veröffentlichungstag der Anmeldung: **15.05.2002**
(21) Anmeldenummer: 01125413.3
(22) Anmeldetag: 31.10.2001
(51) Int. Cl.: C07C 213/08, C07C 219/00, A61K 7/50

(54) **Esterquats**

(30) Priorität: 09.11.2000 DE 10055554
(71) Anmelder: Cognis Ibéria, S.L., 08755 Castellbisal (ES)
(72) Erfinder: Bigorra Llosas, Joaquin, Dr., E-08203 Sabadell (ES); Bonastre Nuria, Gilabert, Dr., ES-08210 Barbera del Vall (ES); Domingo, Marta, DE-08024 Barcelona (ES); Pi Subirana, Rafael, Dr., E-08400 Granollers (ES)
(74) Vertreter: Fabry, Bernd, Dr.

(57) **Zusammenfassung**

Vorgeschlagen werden neue Esterquats, die man erhält, indem man Mischungen von Carbonsäuren und oxidierten Paraffinen mit Alkanolaminen und/oder Diaminen umsetzt und die resultierenden Ester bzw. Amide mit Alkylhalogeniden oder Dialkylsulfaten quaterniert.

## Beschreibung

### Gebiet der Erfindung

Die Erfindung befindet sich auf dem Gebiet der kationischen Tenside und betrifft neue Esterquats, die man durch Kondensation von Carbonsäuren mit oxidierten Paraffinen und Alkanolaminen bzw. Diaminen gewinnt, ein Verfahren zu ihrer Herstellung sowie ihre Verwendung zur Ausrüstung von Textilien.

### Stand der Technik

Unter der Bezeichnung "Esterquats" werden im allgemeinen quaternierte Fettsäuretriethanolaminestersalze verstanden. Es handelt sich dabei um bekannte Stoffe, die man nach den einschlägigen Methoden der präparativen organischen Chemie erhalten kann. In diesem Zusammenhang sei auf die Internationale Patentanmeldung **WO 91/01295** (Henkel) verwiesen, nach der man Triethanolamin in Gegenwart von unterphosphoriger Säure mit Fettsäuren partiell verestert, Luft durchleitet und anschließend mit Dimethylsulfat oder Ethylenoxid quaterniert. Aus der Deutschen Patentschrift **DE 4308794 C1** (Henkel) ist ferner ein Verfahren zur Herstellung fester Esterquats bekannt, bei dem man die Quaternierung von Triethanolaminestern in Gegenwart von geeigneten Dispergatoren, vorzugsweise Fettalkoholen, durchführt. Schließlich ist Gegenstand des europäischen Patentes **EP 0770594 B1** (Cognis) die Herstellung von Esterquats unter Einsatz von Fettsäuren und Dicarbonsäuren. Übersichten zu diesem Thema sind beispielsweise von R.Puchta et al. in **Tens.Surf.Det., 30, 186 (1993),** M.Brock in **Tens.Surf.Det. 30, 394 (1993),** R.Lagerman et al. in **J.Am. Oil.Chem.Soc., 71, 97 (1994)** sowie I.Shapiro in **Cosm.Toil. 109, 77 (1994)** erschienen.

Esterquats finden wegen ihrer Eigenschaft, Textilien und Fasern einen angenehmen Weichgriff zu verleihen, insbesondere auch als Avivagemittel ("Softener") Verwendung. Hierbei nutzt man die Fähigkeit der kationischen Tenside aus, auf die Fasern aufzuziehen und dabei u.a. die statische Aufladung herabzusetzen, also für eine antistatische Ausrüstung zu sorgen. Durch Kombination von Esterquats mit Polyolefinwachsen, wie dies im europäischen Patent **EP 0784666 B1** (Cognis) beschrieben wird, kann der Weichgriff sogar noch verbessert werden, insbesondere wird aber die Friktion herabgesetzt und das Bügeln der so behandelten Textilien deutlich erleichtert. Entsprechende Produkte befinden sich beispielsweise unter der Bezeichnung "Vernell Easy" bereits im Handel.

Nun ist der Einsatz von Mischprodukten stets mit zusätzlichem Aufwand und Lagerhaltung verbunden und wird daher von den Herstellern der Endprodukte wenig geschätzt. Dies geht einher mit der permanenten Forderung nach höherer Leistung, vorzugsweise bei reduzierter Einsatzmenge. Die Aufgabe der vorliegenden Erfindung hat folglich darin bestanden, dieser Forderung gerecht zu werden.

### Beschreibung der Erfindung

Gegenstand der Erfindung sind neue Esterquats, erhältlich, indem man Mischungen von Carbonsäuren und oxidierten Paraffinen mit Alkanolaminen und/oder Diaminen umsetzt und die resultierenden Ester bzw. Amide mit Alkylhalogeniden oder Dialkylsulfaten quaterniert.

Überraschenderweise wurde gefunden, dass der Einbau von oxidierten Paraffinen, die als Ergebnis des Oxidationsprozesses selbst über Carboxylfunktionen verfügen, zu neuen Esterquats führt, die sich bei der textilen Ausrüstung sowohl herkömmlichen Esterquats, als auch bekannten Mischungen von Esterquats mit Polyolefinwachsen überlegen erweisen. Neben einer Verbesserung des Weichgriffes werden sowohl die statische Aufladung als auch die Friktion erniedrigt, wodurch die so behandelten Textilien besonders bügelleicht werden. Im übrigen wird der gewünschte Effekt nunmehr von einem Stoff erfüllt, wo es vorher der Abmischung von zwei Komponenten bedurfte.

### Herstellverfahren

Ein weiterer Gegenstand der Erfindung betrifft ein Verfahren zur Herstellung der neue Esterquats, bei dem man Mischungen von Carbonsäuren und oxidierten Paraffinen mit Alkanolaminen und/oder Diaminen umsetzt und die resultierenden Ester bzw. Amide mit Alkylhalogeniden oder Dialkylsulfaten quaterniert.

### Carbonsäuren

Üblicherweise werden zur Herstellung der Esterquats als Carbonsäuren Fettsäuren eingesetzt, welche der Formel (I) folgen,

**R**^{**1**}**CO-OH** (I)

in der R¹CO für einen linearen oder verzweigten, gesättigten oder ungesättigten Acylrest mit 6 bis 22, vorzugsweise 12 bis 18 Kohlenstoffatomen und 0 und/oder 1 bis 3 Doppelbindungen steht. Typische Beispiele für geeignete Fettsäuren sind Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen oder bei der Reduktion von Aldehyden aus der Roelen'schen Oxosynthese anfallen. Bevorzugt sind technische Fettsäuren mit 12 bis 18 Kohlenstoffatomen wie beispielsweise Kokos-, Palm-, Palmkern- oder Talgfettsäure, sowohl in gehärteteter als auch teilgehärteter (Iodzahlen zwischen 5 und 50) Form.

In einer bevorzugten Ausführungsform der Erfindung werden als Carbonsäuren Mischungen von Fettsäuren und Dicarbonsäuren der Formel (**II**) eingesetzt,

**HOOC-(CH**_{**2**}**)**_{**n**}**COOH** (II)

in der n für Zahlen von 1 bis 10, vorzugsweise 6 bis 8 steht. Typische Beispiele sind Bernsteinsäure, Glutarsäure, Pimelinsäure, Azelainsäure, Dodecandisäure und insbesondere Adipinsäure, weil auf diesem Wege klarlösliche Produkte erhalten werden. Es hat sich als vorteilhaft erwiesen, die Fettsäuren und die Dicarbonsäuren im molaren Verhältnis von 1 : 1 bis 10 : 1, vorzugsweise 3 : 1 bis 7 : 1 einzusetzen.

### Oxidierte Paraffine

Oxidierte Paraffine stellen bekannte Stoffe dar, die beispielsweise durch Einwirkung von Wasserstoffperoxid auf gesättigte, lineare Kohlenwasserstoffe aus der Erdöldestillation erhältlich sind; entsprechende Produkte werden beispielsweise von der BASF AG vertrieben. Typischerweise gelangen oxidierte Paraffine zum Einsatz, die eine Kettenlänge von 10 bis 100, vorzugsweise 20 bis 80 und insbesondere 30 bis 50 Kohlenstoffatomen und eine Säurezahl im Bereich von 5 bis 100, vorzugsweise 20 bis 80 und insbesondere 60 bis 70 aufweisen. In der Regel werden die Carbonsäuren und die oxidierten Paraffine im Gewichtsverhältnis 99 : 1 bis 50 : 50, vorzugsweise 90 : 10 bis 85 : 15 eingesetzt.

### Alkanolamine und Diamine

Als Alkanolamine kommen neben Mono- und Diethanolamin insbesondere Triethanolamin, Methyldiethanolamin sowie deren Gemische in Frage. Alternativ können auch Diamine eingesetzt werden, beispielsweise Ethylendiamin, Propylendiamin, Butylendiamin, Diethylentriamin, Triethylentetraamin sowie deren Gemische, vorzugsweise aber Dimethylaminopropylamin. In diesem Fall kommt es zwar anstelle der Ester- zu einer Amidbindung. Zur Herstellung der quaternierten Ester bzw. Amide, die im übrigen aus der eingangs zitierten Literatur an sich bekannt ist, können die Fettsäuren/oxidierten Paraffine bzw. deren Gemische mit Dicarbonsäuren und die Alkanolamine bzw. Diamine im molaren Verhältnis - bezogen auf die Säurefunktionen - von 1,1 : 1 bis 3 : 1 eingesetzt werden. Im Hinblick auf die anwendungstechnischen Eigenschaften der Esterquats hat sich ein Einsatzverhältnis von 1,2 : 1 bis 2,2 : 1, vorzugsweise 1,5 : 1 bis 1,9 : 1 als besonders vorteilhaft erwiesen. Die bevorzugten Esterquats stellen technische Mischungen von Mono-, Di- und Triestern mit einem durchschnittlichen Veresterungsgrad von 1,5 bis 1,9 dar. Für die Einstellung eines solchen Verhältnisses hat es sich als vorteilhaft erwiesen, die Kondensation in Gegenwart von Hypophosphoriger Säure oder einem entsprechenden Alkalisalz durchzuführen.

### Alkylierungsmittel

Auch die Alkylierung der wie oben beschrieben erhältlichen Ester bzw. Amide kann in an sich bekannter Weise erfolgen. Obschon die Umsetzung auch in Abwesenheit von Lösungsmittel gelingt, empfiehlt sich die Mitverwendung zumindest von geringen Mengen Wasser oder niederer Alkohole, speziell Isopropylalkohol oder Propylenglycol, zur Herstellung von Konzentraten, die typischerweise einen Feststoffgehalt von mindestens 80 Gew.-% aufweisen. Als Alkylierungsmittel kommen Alkylhalogenide, wie beispielsweise Methylchlorid oder Dialkylsulfate, vorzugsweise Dimethylsulfat in Frage. Daneben kann die Quaternierung auch mit Dialkylcarbonaten durchgeführt werden. Üblicherweise werden die Ester bzw. Amide und die Alkylierungsmittel im molaren Verhältnis 1 : 0,90 bis 1 : 1,05, also annähernd stöchiometrisch eingesetzt. Die Reaktionstemperatur liegt gewöhnlich bei 40 bis 80 und insbesondere 50 bis 60 °C. Wie schon in der einleitend genannten Literatur ausführlich beschrieben, kann die Quaternierung auch in Gegenwart von Emulgatoren und/oder Dispergatoren durchgeführt werden.

### Gewerbliche Anwendbarkeit

Der Einsatz der neuen Esterquats führt nicht nur zu einem besonderen Weichgriff, sondern verringert sohl die statische Aufladung als auch die Friktion der Gewebe. Ein weiterer Gegenstand der Erfindung betrifft daher die Verwendung der neuen Esterquats zur Herstellung von Textilbehandlungsmitteln, speziell Mitteln zur Verbesserung der Bügelbarkeit von Textilien, in denen sie in Mengen von 1 bis 80, vorzugsweise 10 bis 50 und insbesondere 20 bis -30 Gew.-% - bezogen auf die Endprodukte - enthalten sein können. Die weiteren in den Endprodukten enthaltenen Stoffe stellen im einfachsten Fall Lösungsmittel, beispielsweise Wasser, Ethanol, Isopropylalkohol, Ethylenglycol, Propylenglycol oder Glycerin dar. Ebenfalls enthalten sein können Emulgatoren und weitere Wachse, bei denen es sich beispielsweise um Polyolefinwachse handeln kann.

In einer besonderen Ausführungsform der Erfindung gelangen Mischungen von Esterquats mit Ester- und Amidbindungen zum Einsatz. Dies kann beispielsweise derart erfolgen, dass die Carbonsäuren mit Mischungen von Alkanolaminen und Diaminen kondensiert und dann quaterniert werden, bzw. dass die Ester und Amide als Vorstufen getrennt synthetisiert und dann gemeinsam quaterniert werden. Natürlich ist es auch möglich, die quaternierten Carbonsäurealkanolaminester und die analogen quaternierten Carbonsäureamidoamine getrennt herzustellen und dann abzumischen, wobei in jedem Fall ein Mischungsverhältnis von 10 : 90 bis 90 : 10, vorzugsweise 25 : 75 bis 75 : 25 und insbesondere 40 : 60 bis 60 : 40 für eine besonders hohe Lagerstabilität, Transparenz und Weichgriff von Vorteil ist. Vorzugsweise kommen solche quaternierten Carbonsäureamidoamine zum Einsatz, die sich von Fettsäuren mit 12 bis 18 Kohlenstoffatomen, vorzugsweise Kokosfettsäure, Talgfettsäure oder Ölsäure, gegebenenfalls in Mischung mit Adipinsäure, und Dimethylaminopropylamin ableiten.

### Beispiel 1.

In einem Rührreaktor wurden 330,6 g Kokosfettsäure, 175 g Adipinsäure, 17,4 g eines oxidierten Paraffins vom Typ Petrolatum SZ50 (Säurezahl 50) - entsprechend 5 Gew.-% bezogen auf die Fettsäure - und 0,75 g Natriumhypophosphit vorgelegt und bei vermindertem Druck von 20 mbar auf 70 °C erhitzt. Anschließend wurden portionsweise 358 g Triethanolamin zugetropft und die Temperatur dabei gleichzeitig auf 120 °C gesteigert. Nach Beendigung der Zugabe wurde der Ansatz auf 160 °C erhitzt, der Druck auf 3 mbar angesenkt und die Mischung über einen Zeitraum von 2,5 h bei diesen Bedingungen gerührt, bis die Säurezahl auf einen Wert unterhalb von 5 abgesunken war. Anschließend wurde die Mischung auf 60 °C abgekühlt, das Vakuum durch Einleiten von Stickstoff gebrochen und 0,6 g Wasserstoffperoxid in Form einer 30 Gew.-%igen wäßrigen Lösung zugegeben. Für die Quaternierung wurden 1,3 Mol des resultierenden Ester in 148 g Isopropylalkohol gelöst und über einen Zeitraum von 1 h mit einer solchen Geschwindigkeit mit 156 g Dimethylsulfat versetzt, dass die Temperatur nicht über 65 °C anstieg. Nach Beendigung der Zugabe ließ man den Ansatz weitere 2,5 h rühren, wobei der Gesamtstickstoffgehalt durch Probennahme regelmäßig überprüft wurde. Die Reaktion wurde beendet, nachdem ein konstanter Gesamtstickstoffgehalt erreicht wurde. Es wurde ein Produkt mit einem Feststoffgehalt von 80 Gew.-% erhalten. Eine 30 Gew.-%ige wäßrige Lösung war unmittelbar nach ihrer Herstellung klar, trübte sich jedoch innerhalb von 14 Tagen leicht.

### Beispiel 2.

Beispiel 1 wurde wiederholt, jedoch 296 g Kokosfettsäure, 154 g Adipinsäure, 52,2 g oxidiertes Petrolatum - entsprechend 15 Gew.-% bezogen auf Kokosfettsäure - und 314 g Triethanolamin verestert und dann quaterniert. Eine 30 Gew.-%ige wäßrige Lösung war unmittelbar nach ihrer Herstellung klar, trübte sich jedoch innerhalb von 14 Tagen leicht.

### Beispiel 3.

Beispiel 1 wurde wiederholt, jedoch 330,6 g Kokosfettsäure, 169 g Adipinsäure, 17,4 g oxidiertes Paraffin vom Typ AAX3565 mit einer Säurezahl von 65 - entsprechend 5 Gew.-% bezogen auf Kokosfettsäure - und 344,5 g Triethanolamin verestert und dann quaterniert. Eine 30 Gew.-%ige wäßrige Lösung war auch nach 14tägiger Lagerung klar.

### Beispiel 4.

Beispiel 1 wurde wiederholt, jedoch 296 g Kokosfettsäure, 175 g Adipinsäure, 52,2 g oxidiertes Paraffin vom Typ AAX3565 mit einer Säurezahl von 65 - entsprechend 15 Gew.-% bezogen auf Kokosfettsäure - und 358 g Triethanolamin verestert und dann quaterniert. Eine 30 Gew.-%ige wäßrige Lösung war auch nach 14tägiger Lagerung klar.

### Beispiel 5.

Beispiel 1 wurde wiederholt, jedoch 950 g teilgehärtete Talgfettsäure, 50 g oxidiertes Petrolatum SZ50, 290,5 g Triethanolamin und 1 g Natriumhypophosphit verestert. 1167 g des resultierenden Esters wurden in 155 g Isopropylalkohol gelöst und dann mit 225 g Dimethylsulfat quaterniert. Es wurde ein Produkt mit einem Trockenrückstand von 90 Gew.-% erhalten, welches in 30 Gew.-%iger wässriger Verdünnung auch nach 14tägiger Lagerung noch klar war.

### Beispiel 6.

Beispiel 1 wurde wiederholt, jedoch 850 g teilgehärtete Talgfettsäure, 150 g oxidiertes Paraffin vom Typ AAX3565 mit einer Säurezahl von 65, 250,5 g Triethanolamin und 1 g Natriumhypophosphit verestert. 1174 g des resultierenden Esters wurden in 153 g Isopropylalkohol gelöst und dann mit 203 g Dimethylsulfat quaterniert. Es wurde ein Produkt mit einem Trockenrückstand von 90 Gew.-% erhalten, welches in 30 Gew.-%iger wässriger Verdünnung auch nach 14tägiger Lagerung noch klar war.

### Beispiel 7.

Beispiel 1 wurde wiederholt, jedoch 334,4 g Ölsäure 132 g Adipinsäure, 17,6 g oxidiertes Paraffin vom Typ AAX3565 mit einer Säurezahl von 65, 270 g Triethanolamin und 0,7 g Natriumhypophosphit verestert. 680g des resultierenden Esters wurden in 99 g Isopropylalkohol gelöst und dann mit 206 g Dimethylsulfat quaterniert. Es wurde ein Produkt mit einem Trockenrückstand von 90 Gew.-% erhalten. Das Esterquat wurde mit einem methylquaternierten Kondensationsprodukt von Kokosfettsäure und Dimethylaminopropylamin im Gewichtsverhältnis 7:3 abgemischt. Eine 30 Gew.-%ige wässriger Verdünnung der Mischung war auch nach 4wöchiher Lagerung noch klar und verlieh einen besonders vorteilhaften Weichgriff.

## Patentansprüche

1. Esterquats, dadurch erhältlich, dass man Mischungen von Carbonsäuren und oxidierten Paraffinen mit Alkanolaminen und/oder Diaminen umsetzt und die resultierenden Ester bzw. Amide mit Alkylhalogeniden oder Dialkylsulfaten quaterniert.

2. Verfahren zur Herstellung von Esterquats, bei dem man Mischungen von Carbonsäuren und oxidierten Paraffinen mit Alkanolaminen und/oder Diaminen umsetzt und die resultierenden Ester bzw. Amide mit Alkylhalogeniden oder Dialkylsulfaten quaterniert.

3. Verfahren nach Anspruch 2, **dadurch gekennzeichnet, dass** man als Carbonsäuren Fettsäuren der Formel (I) einsetzt,
**R**^{**1**}**CO-OH (I)**
in der R¹CO für einen linearen oder verzweigten, gesättigten oder ungesättigten Acylrest mit 6 bis 22 Kohlenstoffatomen und 0 und/oder 1 bis 3 Doppelbindungen steht.

4. Verfahren nach Anspruch 2, **dadurch gekennzeichnet, dass** man als Carbonsäuren Mischungen von Fettsäuren und Dicarbonsäuren der Formel (**II**) einsetzt,
**HOOC-(CH**_{**2**}**)**_{**n**}**COOH (II)**
in der n für Zahlen von 1 bis 10 steht.

5. Verfahren nach mindestens einem der Ansprüche 2 bis 4, **dadurch gekennzeichnet, dass** man die Fettsäuren und die Dicarbonsäuren im molaren Verhältnis von 1 : 1 bis 10 : 1 einsetzt.

6. Verfahren nach mindestens einem der Ansprüche 2 bis 5, **dadurch gekennzeichnet, dass** man oxidierte Paraffine einsetzt, die eine Kettenlänge von 10 bis 100 Kohlenstoffatome und eine Säurezahl im Bereich von 5 bis 100 aufweisen.

7. Verfahren nach mindestens einem der Ansprüche 2 bis 6, **dadurch gekennzeichnet, dass** man die Carbonsäuren und die oxidierten Paraffine im Gewichtsverhältnis 99 : 1 bis 50 : 50 einsetzt.

8. Verfahren nach mindestens einem der Ansprüche 2 bis 7, **dadurch gekennzeichnet, dass** man als Alkanolamine Triethanolamin, Methyldiethanolamin oder deren Gemische sowie als Diamine Ethylendiamin, Propylendiamin, Butylendiamin, Diethylentriamin, Triethylentetraamin, Dimethylaminopropylamin sowie Gemische von Alkanolaminen und/oder Diaminen einsetzt.

9. Verwendung von Esterquats nach Anspruch 1 zur Herstellung von Textilbehandlungsmitteln.

10. Verwendung von Esterquats nach Anspruch 1 zusammen mit quaternierten Carbonsäureamidoaminen zur Herstellung von Textilbehandlungsmitteln.
